# EUROPEAN PATENT APPLICATION

(11) **EP 2 599 476 A1**
(43) Date of publication of application: **05.06.2013**
(21) Application number: 13156622.6
(22) Date of filing: 03.04.2009
(51) Int. Cl.: A61K 9/127, A61K 38/48, A61P 13/10

(54) **Treatment of bladder dysfunction using liposomal botulinum toxin**

(30) Priority: 04.04.2008 US 42536 P; 31.10.2008 US 110266 P
(62) Divisional of application: 09747070.2
(71) Applicant: Lipella Pharmaceuticals, Inc., Pittsburgh, PA 15217 (US)
(72) Inventor: Chancellor, Michael B, Pittsburgh, PA Pennsylvania 15217 (US); Kaufman, Jonathan H, Pittsburgh, PA Pennsylvania 15217 (US)
(74) Representative: Bassett, Richard Simon

(57) **Abstract**

Liposomes are used for intravesical drug delivery, especially delivery of botulinum toxin (BoNT) to help improve lower urinary tract symptoms by decreasing bladder irritation and frequency. The system uses a lower and safer dose of BoNT with lower risk of urinary retention. A simple instillation of liposome-BoNT as a liquid formulation into the bladder, in a typical volume of 30-60 ml, will achieve efficacy similar to that currently achieved with cystscopic needle injection of BoNT. The dose may be lower than that done by injection, thereby causing significantly less risk of urinary retention. Liposome-BoNT can protect the BoNT from bladder and urine breakdown. Liposome-BoNT instillation is more comfortable for the patients and allows many more doctors' offices to offer this form of treatment that would otherwise be restricted to doctors skilled and certified in cystoscopic BoNT injection.

## Description

### Field of the Invention

This invention is generally in the field of treatments for bladder dysfunction, especially refractory overactive bladder.

### Background of the Invention

Urinary incontinence, or bladder dysfunction, is loss of bladder control. Symptoms can range from mild leaking to uncontrollable wetting. It can happen to anyone, but it becomes more common with age. Most bladder control problems happen when muscles are too weak or too active. If the muscles that keep the bladder closed are weak, there can be urine leakage when sneezing, laughing or lifting a heavy object. This is stress incontinence. If bladder muscles become too active, there is a strong urge to go to the bathroom when there is little urine in the bladder. This is urge incontinence or overactive bladder. There are other causes of incontinence, such as prostate problems and nerve damage.

Treatment depends on the type of problem. It may include simple exercises, medicines, special devices or procedures prescribed by a doctor, or surgery.

Intravesical therapies have been a mainstay in treatment for many years (Parkin, et al., Urology 49, 105-7 (1997). Intravesical pharmacotherapy provides high local drug concentrations in the bladder, low risk of systemic side effects and eliminates the problem of low levels of urinary excretion with orally administered agents. A standard instillation time of 30 min has been tested with excellent tolerability in patients. Clinically, dimethylsulfoxide (DMSO) (Rimso-50) is the only FDA approved intravesical treatment for painful bladder syndrome/interstitial cystitis (PBS/IC), believed to have anti-inflammatory properties and mast cell stabilizing effects (Sun and Chai, BJU Int 90,381-5 (2002). However success rates of DMSO are generally modest. Bladder epithelium relies primarily on the presence of a surface glycosaminoglycan (GAG) layer and the structural integrity of cell-cell contact, namely tight junctions, to maintain impermeability to toxic urinary wastes (Parsons, et al., Science 208, 605-7 (1980). When this barrier is damaged, leakage of urine components into the underlying bladder layers initiates the irritative changes in the bladder leading to the stimulation of sensory nerve fibers and the pain, urgency and frequency symptoms(Lavelle, et al., Am J Physiol Renal Physiol 283, F242-53 (2002). The urothelium and GAG also presents a significant barrier for effective intravesical drug delivery.

Even more significant is overactive bladder (OAB) which affects 17% of men and women in the United States with increasing incidence with aging, and has an estimated economic burden of $16.4 billion. Many experts believe that 25% with OAB will seek medical therapy. Conservatively, it has been estimated that 25% of those 4 Million OAB patients who seek treatment will fail oral pharmacotherapy and seek therapy for refractory OAB.

In the last several years, several new antimuscarinic medications and improved formulations for OAB have emerged. There are six FDA approved OAB antimuscarinic agents in 2008 with global sales of $2B per year. However, fewer than 20% of patients remain on antimuscarinic therapy due to limited efficacy and adverse effects, such as dry mouth, constipation and cognitive dysfunction. Two procedures often implemented as second-line therapy for antimuscarinic refractory patients are the FDA approved sacral nerve stimulation (SNS) (Interstim, Medtronics Inc.) and intra-detrusor injections of BoNT which is in Phase II FDA trials for refractory OAB (Botox, Allergan Inc.,). Several clinical trials are currently being conducted where botulinum toxin is administered. Botulinum toxin has been shown to be helpful to treat refractory overactive bladder (OAB), yet it requires a cystoscopic procedure to directly inject the toxin into the bladder wall. Since the toxin is introduced into the bladder detrusor muscle and can weaken the bladder contractility, up to 43% of patients may develop urinary retention.

The pharmaceutical industry has also shown significant interest in developing therapies for urinary urgency and frequency associated with interstitial cystitis. Most recently, these therapies have included bacillus Calmette-Guerin (BCG), resiniferatoxin (RTX), hyaluronic acid (Cystistat), sodium hyaluronate (SI-7201), and sacral nerve stimulation devices.

There is a need to be able to simply instill botulinum toxin (BoNT) without the need for needle injection. Moreover, there is a need to deliver BoNT to the bladder urothelial lining and sensory nerve terminal at lower concentrations that may help to improve the lower urinary symptoms without causing retention and/or bladder muscle weakness.

There is also need in other urologic conditions such as urinary tract infection and bladder cancer for localized intravesical drug delivery with longer duration of drug contact in the bladder.

There are no commercially available sustained drug delivery systems for bladder dysfunction. Others have attempted to place a balloon reservoir or matrix material with drugs into the bladder for sustained drug release but these mechanical methods cause irritation, pain and can cause obstruction by blockage of bladder neck outlet because of its size and shape.

Limited clinical experience with intravesical BoNT instillation has been unsuccessful so far. Possible reasons underlying the lack of efficacy from BoNT instillation in the bladder includes degradation by proteases in urine, dilution in urine or poor uptake into the urothelium from the BoNT solution instilled into the bladder lumen.

It is therefore an object of the present invention to provide a method and compositions for delivery of drugs by instillation into the bladder for treatment of OAB and other disorders of the bladder.

### Summary of the Invention

Liposomes are used for intravesical drug delivery, especially delivery of BoNT to help improve lower urinary tract symptoms by decreasing bladder irritation and frequency. The system uses a lower and safer dose of BoNT with lower risk of urinary retention, than injection.

A simple instillation of liposome-BoNT as a liquid formulation into the bladder, in a typical volume of 30-60 ml, achieves efficacy similar to that currently achieved with cystscopic needle injection of BoNT. The dose may be lower than that done by injection, thereby causing significantly less risk of urinary retention. Liposome-BoNT can protect the BoNT from bladder and urine breakdown.

Examples demonstrate the efficacy of the formulations.

### Detailed Description of the Invention

### I. Liposome-Drug Formulations

Liposome encapsulation should solve the problems with poor absorption after instillation. Liposomes encapsulation of BoNT can protect BoNT from degradation in urine and allow unhindered absorption across the urothelium from liposomes adhering to the bladder surface. Since BoNT is entrapped inside the liposomes, it is not vulnerable to dilution by urine and localized concentration of BoNT at liposome surface can be high enough to hasten the entry of leached BoNT from liposomes adhering to the surface of bladder lumen.

### A. Liposomes

Liposomes (LPs) are spherical vesicles, composed of concentric phospholipid bilayers separated by aqueous compartments. LPs have the characteristics of adhesion to and creating a molecular film on cellular surfaces. Liposomes are lipid vesicles composed of concentric phospholipid bilayers which enclose an aqueous interior (Gregoriadis, et al., Int J Pharm 300, 125-30 2005; Gregoriadis and Ryman, Biochem J 124, 58P (1971)). The lipid vesicles comprise either one or several aqueous compartments delineated by either one (unilamellar) or several (multilamellar) phospholipid bilayers (Sapra, et al., Curr Drug Deliv 2, 369-81 (2005)). The success of liposomes in the clinic has been attributed to the nontoxic nature of the lipids used in their formulation. Both the lipid bilayer and the aqueous interior core of liposomes can serve the purpose of treatment. Liposomes have been well studied as carrier of toxins for enhancing their efficacy at lower doses (Alam, et al., Mol Cell Biochem 112, 97-107 1992; Chaim-Matyas, et al., Biotechnol Appl Biochem 17 (Pt 1), 31-6 1993; de Paiva and Dolly, FEBS Lett 277, 171-4 (1990); Freitas and Frezard, Toxicon 35, 91-100 (1997); Mandal and Lee, Biochim Biophys Acta 1563, 7-17 (2002)).

Liposomes have the ability to form a molecular film on cell and tissue surfaces and are currently being tested as possible therapeutic agents to promote wound healing and healing dry eye as a tear substitute. Clinical studies have proven the efficacy of liposomes as a topical healing agent (Dausch, et al., Klin Monatsbl Augenheilkd 223, 974-83 (2006); Lee, et al., Klin Monatsbl Augenheilkd 221, 825-36 (2004)). Liposomes have also been used in ophthalmology to ameliorate keratitis, corneal transplant rejection, uveitis, endophthalmitis, and proliferative vitreoretinopathy (Ebrahim, et al., 2005; Li, et al., 2007).

Liposomes have been widely studied as drug carriers for a variety of chemotherapeutic agents (approximately 25,000 scientific articles have been published on the subject) (Gregoriadis, N Engl J Med 295, 765-70 (1976); Gregoriadis, et al., Int J Pharm 300, 125-30 (2005)). Water-soluble anticancer substances such as doxorubicin can be protected inside the aqueous compartment(s) of liposomes delimited by the phospholipid bilayer(s), whereas fat-soluble substances such as amphotericin and capsaicin can be integrated into the phospholipid bilayer (Aboul-Fadl, Curr Med Chem 12, 2193-214 (2005); Tyagi, et al., J Urol 171, 483-9 (2004)), Topical and vitreous delivery of cyclosporine was drastically improved with liposomes (Lallemand, et al., Eur J Pharm Biopharm 56,307-18 2003). Delivery of chemotherapeutic agents lead to improved pharmacokinetics and reduced toxicity profile (Gregoriadis, Trends Biotechnol 13, 527-37 (1995); Gregoriadis and Allison, FEBS Lett 45, 71-4 1974; Sapra, et al., Curr Drug Deliv 2, 369-81 (2005)). More than ten liposomal and lipid-based formulations have been approved by regulatory authorities and many liposomal drugs are in preclinical development or in clinical trials (Barnes, Expert Opin Pharmacother 7, 607-15 (2006); Minko, et al., Anticancer Agents Med Chem 6, 537-52 (2006)). Fraser, et al. Urology, 2003; 61: 656-663 demonstrated that intravesical instillation of liposome enhanced the barrier properties of dysfunctional urothelium and partially reversed the high micturition frequency in a rat model of hyperactive bladder induced by breaching the uroepithelium with protamine sulfate and thereafter irritating the bladder with KCl. Tyagi et al. J Urol., 2004; 171; 483-489 reported that liposomes are a superior vehicle for the intravesical administration of capsaicin with less vehicle induced inflammation in comparison with 30% ethanol. The safety data with respect to acute, subchronic, and chronic toxicity of liposomes has been assimilated from the vast clinical experience of using liposomes in the clinic for thousands of patients. The safe use of liposomes for the intended clinical route is also supported by its widespread use as a vehicle for anticancer drugs in patients.

### B. Botulinum toxin (BoNT) and Other drugs for instillation

Botulinum neurotoxin, which is produced by *Clostridium botulinum,* is regarded as the most potent biological toxin known to man (Smith & Chancellor, J Urol, 171: 2128 (2004). BoNT has been used effectively for different conditions with muscular hypercontraction. Among seven immunologically distinct neurotoxins (types A to G), BoNT-A is the most commonly used botulinum toxin clinically, In the last few years, BoNT-A and BoNT-B have been used successfully for the treatment of spinal cord injured patients with neurogenic bladder hyperactivity using intradetrusor BoNT-A injection at multiple sites (Schurch et al., 2000).

Effects on ACh and Norepinephrine Release Inhibition: BoNT is been known to exert effects by inhibiting ACh release at the neuromuscular junction as well as autonomic neurotransmission. After intramuscular injection of BoNT temporary chemodenervation and muscle relaxation can be achieved in skeletal muscle as well as in smooth muscle (Chuang & Chancellor, J Urol. 176(6 Pt 1):2375-82 (2006)). Smith et al. (J Urol, 169: 1896 (2003)) found that BoNT injection into the rat proximal urethral sphincter caused marked decreases in labeled norepinephrine at high but not at low electrical field stimulation, indicating that BoNT inhibits norepinephrine release at autonomic nerve terminals.

Effects on Sensory Mechanism Inhibition: There has been increasing evidence to support the notion that BoNT also inhibits afferent neurotransmission in the bladder (Chuang et al., J Urol 172, 1529-32 (2004); Khera et al., Neurochem Int, 45: 987 (2004)). It has been shown to inhibit the release of neuropeptides, glutamate and adenosine triphosphate, which are mediators of painful sensation (Cui et al., Pain, 107: 125 (2004)). Similar effects were observed in an acetic acid induced bladder overactivity model. Decreased levels of the sensory receptors P2X3 and transient receptor potential vanilloid 1 (TRPV1) receptors in suburothelial nerve fibers associated with decreased urgency following intradetrusor injections of BoNT have been found in human detrusor overactivity (Apostolidis et al., J Urol, 174: 977 2005).

The target protein for BoNT is an integral membrane protein which resides in a lipid environment. Liposomes can enhance the activity of metalloproteases such as BoNT by allowing stronger adhesion to the urothelium. Cystoscope guided injections is the current standard practice in the clinic for administering BoNT to bladder. In recent years, studies have assessed the potential of intravesical instillation of BoNT in animals models of bladder irritation (Khera, et al., Urology 66, 208-12 (2005)). Previous reports in the literature suggest that metalloproteolytic activity of the BoNT specific for VAMP is strongly enhanced by the presence of lipid membranes. This effect provides an explanation for the fact that these neurotoxins are more active on VAMP inserted into lipid vesicles and in neurons than on the isolated VAMP molecule. This lipid-enhancing effect is brought about by lipids. This research strongly supports the rational for using liposome as a delivery vehicle for BoNT instillation.

Other drugs that can be instilled into the bladder may also be delivered using the liposome delivery system, especially those having systemic side effects that are avoided by local delivery.

Suitable drugs or active agents that can be delivered using the disclosed liposome delivery system include, but are not limited to, cancer therapeutics, immunomodulators, analgesics, anti-inflammatory agents, antihistamines, endorphins, prostaglandine, canaboid TRP receptors, peptides, proteins, and antibodies, plasmids, naked DNA, viral vectors, RNA, siRNA, amino acids; hyaluronic acid; pentosan polysulfate sodium, beta 3 receptor agonists and antagonists, Ghrelin receptor agonists and antagonists and local anesthetics such as lidocaine.

Exemplary cancer therapeutics include cisplatin, carboplatin, mitomycin, oxaliplatin, mechlorethamine, cyclophosphamide, chlorambucil, vincristine, paclitaxel, vinblastine, doxorubicin, methotrexate, vinorelbine, vindesine, taxol and derivatives thereof, irinotecan, topotecan, amsacrine, etoposide, etoposide phosphate, teniposide, epipodophyllotoxins, trastuzumab (HERCEPTIN®), cetuximab, and rituximab (RITUXAN® or MABTHERA®), bevacizumab (AVASTIN®), and combinations thereof.

Exemplary immunomodulators include interferon and bacille Calmette-Guérin.

The disclosed drug delivery compositions can also be used to deliver suitable drugs to treat interstitial cystitis, painful bladder syndrome, overactive bladder, bladder cancer, prostate cancer, and urinary tract infections caused by bacteria, fungus, or viruses.

### II. Methods of Manufacturing

### Manufacturing of liposomes:

Methods of manufacturing of the liposomes are described in the literature cited above and are well known.

In one embodiment, aqueous liposome suspensions are produced by microfluidization. The end product may be subject to a series of stability problems such as aggregation, fusion and phospholipid hydrolysis (Nounou, et al., Acta Pol Pharm 62, 381-91 (2005)).

The liposomal product must possess adequate chemical and physical stability before its clinical benefit can be realized (Torchilin, Adv Drug Deliv Rev 58,1532-55 (2006)). In a preferred embodiment, dehydrated liposomes are formed from a homogenous dispersion of phospholipid in a tert-butyl alcohol (TBA)/water cosolvent system. The isotropic monophasic solution of liposomes is freeze dried to generate dehydrated liposomal powder in a sterile vial. The freeze drying step leaves empty lipid vesicles or dehydrated liposomes after removing both water and TBA from the vial. On addition of physiological saline, the lyophilized product spontaneously forms a homogenous liposome preparation (Amselem, et al., J Pharm Sci 79, 1045-52 (1990); Ozturk, et al., Adv Exp Med Biol 553, 231-42 (2004)), Low lipid concentrations works ideally for this method because lipid and TBA ratio is the key factor affecting the size and the polydispersity of resulting liposome preparation.

### Preparation for Liposomal BoNT:

In a preferred embodiment, Liposomal BoNT ("LPA-08") is prepared by a dehydration-rehydration method with slight modifications. Liposomes prepared in the previous step are hydrated with a solution of BoNT in water for injection (50 units/ml) at 37°C. Then the mixture is incubated for 2h at the temperature of 37°C using water bath to form oligolamellar hydration liposomes. Mannitol is added to the final mixture at a concentration of 0.5%, 1%, 2.5% and 5% mannitol (w/v), respectively before freezing in acetone-dry ice bath. Mannitol acts as a cryoprotectant in the freeze-drying process. The frozen mixture is lyophilized at -40°C and 5 milibar overnight. The lyophilized cake is resuspended with saline to the desired final concentration of BoNT. The free BoNT is removed from entrapped BoNT by centrifugation at 12,000×g for 30 min using ultracentrifuge. After washing three times, the precipitates are again resuspended in saline.

Formulation of potent bacterial toxins into liposomes requires a meticulous approach. BoNT can not be exposed to organic solvents that are generally used in manufacture of liposomes. Examples were done using the thin film hydration method and the lipid dipalmitoyl phosphatidylcholine (DPPC). Briefly, a solution of DPPC in chloroform was first evaporated under thin stream of nitrogen in a round bottom flask. The lipid film was dried overnight under vacuum. Dried lipids were then hydrated with aqueous BoNT solution.

Optimal results are obtained by carefully controlling the BoNT to lipid ratio, as demonstrated below in Example 2. The optimal ratio and lipid composition can be determined using routine experimentation, as demonstrated by this study.

### III. Liposomal BoNT Delivery

Liposome-BoNT instillation is more comfortable for the patients and allows many more doctors' offices to offer this form of treatment than would otherwise be restricted to doctors skilled and certified in cystoscopic BoNT injection. BoNT is a large molecule and does not penetrate cell layers to reach muscle and nerve terminal. Instillation of BoNT without the use of liposomes would otherwise require caustic agents such as protamine that could damage the bladder lining. This is not approved by the FDA and can cause bladder pain and damage. Instillation of BoNT may bring down the cost of treatment for patients of refractory overactive bladder. Various studies reported from different labs have assessed the potential of intravesical instillation of BoNT in animal models of bladder irritation (Chuang, et al., 2004; Khera, et al., 2005). However, limited clinical experience with intravesical instillation of BoNT has been unsuccessful. Liposome encapsulation solves the problems with poor absorption after instillation. Since BoNT is entrapped inside the liposomes, it is not vulnerable to dilution by urine and localized concentration of BoNT at liposome surface is high enough to hasten the passive diffusion of leached BoNT from liposomes adherent on the bladder surface. The lipid barrier of liposomes can also prevent the access of proteases and proteinases in urine from cleaving the BoNT before it is absorbed by the bladder.

Methods of instillation are known. See, for example, Lawrencia, et al., Gene Ther 8, 760-8 (2001); Nogawa, et al., J Clin Invest 115, 978-85 (2005); Ng, et al., Methods Enzymol 391, 304-13 2005; Tyagi, et al., J Urol 171, 483-9 (2004); Trevisani, et al., J Pharmacol Exp Ther 309, 1167-73 (2004); Trevisani, et al., Nat Neurosci 5, 546-51 (2002)); (Segal, et al., 1975). (Dyson, et al., 2005). (Batista, et al., 2005; Dyson, et al., 2005).

The volume of liposome-BoNT is important in the efficacy of delivery, as demonstrated by example 1, below. Routine experimentation can be used to optimize the delivery volume.

The disclosed liposomes can also be used to instill therapeutic agents to other sites such as the urinary tract including the urethra, bladder, ureter and intrarenal collecting system; gynecological sites such as vaginal, uterus, fallopian tube; gastrointestinal sites including mouth, esophagus, stomach, intestine, colon, rectum, anus; and the outer or inner ear; skin, nose.

The present invention will be further understood by reference to the following non-limiting examples.

### Example 1: Effect of bladder distension on the absorption of liposomal BoNT after instillation.

### Materials and Methods

The effect of bladder distension on the absorption of liposomal BoNT after instillation was determined by instilling the same dose of liposomal BoNT in increasing volumes. Rats were instilled with liposomes made with the same compositions of lipids and BoNT (0.5mg of lipid for each unit of BoNT) in different volume of instillations (0.5, 0.55, 0.6ml). Liposomes were instilled into rat bladder under halothane anesthesia with a dwell time of 30min; animals were then allowed to recover, given food and water and housed in cages. 24 hours later under urethane anesthesia (dose1.0 g/kg body weight), transurethral open cystometry was performed on treated rats by infusing saline at the rate of 0.04ml/min. After getting a normal baseline for one hour acetic acid (0.25%) in saline was infused to induce bladder irritation. Seven days after BoNT treatment with different volumes of instillation, the effect of bladder distension was assessed by continuous transurethral cystometry under urethane anesthesia.

### Results

Rats instilled with a standard volume of 0.5ml, showed regular CMG on reflex voiding induced by constant infusion of saline at the rate of 0.08ml/min. However, rats infused with the same dose of liposomal BoNT in 10-20% higher volume of instillation (0.55ml or 0.6ml volume of instillation) showed features of overflow continence under saline cystometry.

Low volume of instillation restricts the effect of BoNT to urothelium only, but a 10-20% increase in volume of instillation drastically reduces the barrier to absorption and allows BoNT to reach the detrusor muscle after instillation. Rats instilled with high volumes of liposomal BoNT showed urinary retention in awake condition and overflow incontinence under anaesthetized condition to indicate strong suppression of reflex voiding. It is interesting to note that overflow incontinence under anaesthetized condition reflects a desirable feature for drugs acting on the afferent arm of micturition reflex. Overflow incontinence under anaesthetized condition translates into reduced afferent input under awake condition.

BoNT is a large molecule with a molecular weight of 150 kD, so diffusion can be largely ruled out as a mechanism of absorption. Endocytosis is a more likely mechanism in bladder absorption. Previous studies have shown that endocytosis in umbrella cells of urothelium increases with external stimuli such as hydrostatic pressure. The rates of endocytosis and exocytosis during bladder filling are such that the net effect is to add membrane and increase the surface area of urothelium to accommodate bladder stretching. Therefore there is likely to be more endocytotic activity following bladder stretching to cause improved bladder uptake of BoNT.

### Example 2: Effect of Ratio of Lipid to BoNT

For optimum efficacy of BoNT, the lipid and toxin has to be in the optimum ratio.

### Materials and Methods

Liposomal BoNT were prepared with differing ratios of lipid, keeping the ratio of BoNT fixed. For example, starting with 25 IU of BoNT, rats were instilled with liposomes made with same compositions of lipids and BoNT (0.5mg of lipid for each unit of BoNT) in different volumes under halothane anesthesia. The intravesical dose of BoNT was kept constant and the lipid concentration was varied to determine the optimum toxin: lipid ratio. Efficacy of liposomal BoNT was compared against free BoNT in saline solution. The ability to blunt acetic acid induced bladder irritation 7 days after instillation was tested in halothane anaesthetized rats. The 7 days interval between instillation and evaluation of efficacy was chosen based on published studies (Chuang, et al., 2004).

### Results

An optimal 1:0.5 ratio of toxin to lipid is effective in enhancing the efficacy of BoNT after intravesical instillation. Reduced efficacy at higher lipid ratio may be due to very slow release of BoNT from multilamellar liposomes leading to ineffective uptake of BoNT into bladder.

### Example 3: Evaluation of urodynamic and immunohistochemical effects of intravesical BoNT-A liposomal delivery in acetic acid induced bladder hyperactivity in rats.

### Materials and Methods

**Preparation of liposomes (LPs), Botulinum toxin A (BoNT-A), and Lipotoxin (LPs+BoNT-A): LPs** (10 mg, **Lipoid)** dispersed in physiological saline (1 ml), where the dispersion is in liposomal form. BoNT-A dissolved in physiological saline (1 ml, 20u/ml in saline, Allergan, Irvine, CA). LPs encapsulating BoNT-A (referred to as Lipotoxin) were prepared by a modified dehydration-rehydration vesicles method that loads 20 units of BoNT-A into 10mg of LPs dispersion (1 ml) (Gregoriadis, et al., Methods 19, 156-62 1999). Dose of BoNT-A remained same in different animal groups.

**Cystometrogram (CMG):** All experimental procedures were performed on female Sprague-Dawley rats (220-280 gm) and reviewed and approved by the Institutional Animal Care and Use Committee before the study began. Animals were anesthetized by subcutaneous injection of urethane (1.2 g/kg). PE-50 tubing was inserted into the bladder through the urethra and connected via a three-way stopcock to a pressure transducer and to a syringe pump for recording intravesical pressure and for infusing solutions into the bladder. On day 1, a control CMG was performed by filling the bladder with saline (0.08 ml/min) to elicit repetitive voiding. The amplitude, pressure threshold (PT), pressure baseline (PB) and intercontraction interval (ICI) of reflex bladder contractions were recorded. Measurements in each animal represented the average of 3 to 5 bladder contractions.

On day 8, after a baseline measurement was established during saline infusion, we infused 0.3% acetic acid (AA) into the bladder at 0.08 ml/min to acutely promote bladder hyperactivity. 3 to 5 bladder contractions were measured after 30 min of infusion.

**Instillation of drugs:** On day 1 after baseline CMG, PE-50 tubing (Clay-Adams, Parsippany, NJ) was tied in place by a ligature around the urethral orifice under halothane anesthesia. The bladder was emptied of urine, and filled with LPs, BoNT-A or Lipotoxin for 1 hour through the catheter.

**Transcardiac perfusion:** On day 8 after the CMG study, some animals (N=6, for each group) were deeply anesthetized and sacrificed via transcardiac perfusion, first with Krebs buffer followed by 4% paraformaldehyde fixative. The animals were then dissected to harvest the bladder.

**Histology:** The bladders tissues for histology were fixed in 4% paraformaldehyde in phosphate-buffered saline (PBS) for 4 hours, and then in 30% sucrose in PBS overnight. Samples for histology were embedded in paraffin, cut in 10 µm thick pieces and stained with H & E. The AA-induced inflammatory reaction was graded by a score of 0 -3 as follows: 0, no evidence of inflammatory cell infiltrates or interstitial edema; 1, mild (few inflammatory cell infiltrates and little interstitial edema); 2, moderate (moderate amount of inflammatory cell infiltrates and moderate interstitial edema); 3, severe (diffuse presence of large amount of inflammatory cell infiltrates and severe interstitial edema.

**Immunofluorescence microscopy for CGRP and SNAP-25:** Alternatively, samples were frozen and mounted in Tissue-Tek OCT mounting medium (Sakura Finetek, Torrance, CA, USA); 10µm thick longitudinal sections were cut on a cryostat and mounted on SuperFrost slides. Sections were fixed by immersion in acetone for 15 minutes, then washed in phosphate buffered saline (PBS) and blocked endogenous peroxidase activity by incubating the slides in 0.3% H₂O₂ solution in PBS for 10 minutes. After further washing in PBS the sections were incubated in Image-iT^{TW} fix signal enhancer (Molecular Probes, Invitrogen) for 1 hour. For CGRP immunostaining, slides were then stained with goat anti-CGRP polyclonal antibody (Santa Cruz, 1:50 dilution) at +4°C for 48 hrs. The following day sections were then washed in PBS and incubated with donkey anti-goat IgG FITC (Santa Cruz, 1:2000 dilution) for 1 hour. Sections were washed and mounted in SlowFade antifade gold reagent ((Molecular Probes, Invitrogen). For SNAP-25 immunostaining, slides were then stained with mouse anti-SNAP-25 monoclonal antibody (AbD, NC, USA, 1:2000 dilution) at +4°C over night. The following day sections were then washed in PBS and incubated with goat anti-mouse IgG Alexa Fluor 488 (Molecular Probes, Invitrogen, 1:2000 dilution) for 1 hour. To detect muscle fibers, sections were counterstained with tetramethylrhodamine isothiocyanate (TRITC)-labelled phalloidin (Sigma, 1:2000 dilution), washed and the mounted in SlowFade antifade gold reagent ((Molecular Probes, Invitrogen). The slides were examined with a fluorescence microscope to record the image.

**Western blot analysis for SNAP-25 expression:** Some animals (N=6, for each group) were deeply anesthetized and sacrificed without transcardiac perfusion. The bladders were removed for western blot analysis of SNAP-25 expression according to the standard protocol (Amersham Biosciences). The samples were homogenized in protein extraction solution (T-PER; Pierce Biotechnology) prior to sonication and purification. The amount of total protein was measured with the Bradford protein assay method (Bio-Rad Laboratories, Hercules, CA). SDS-polyacrylamide gel electrophoresis (PAGE) was performed using the buffer system of Laemmli. Briefly, an aliquot of the extracts equivalent to 30µg protein was loaded onto 8% polyacrylamide gel, electrophoresed at a constant voltage of 100V for 1h and transferred to Hybond-P PVDF Membrane (Amersham Biosciences). The membrane was blocked with blocking agent and then immunoblotted overnight at +4C with mouse anti-SNAP-25 monoclonal antibody (AbD, NC, USA, 1:500 dilution) and mouse anti-β-actin monoclonal antibody (Rockland, Gilbertsville, USA, 1:2000 dilution) After wash, the membrane was incubated with secondary antibody using 5% defatted milk powder in TBS for 2 hr at room temperature using a horseradish peroxidase-linked anti-rabbit or anti-mouse immunoglobulin G. Western blots were visualized by enhanced chemiluminescence (ECL) detection system (Amersham Biosciences). The amount of β-actin was also detected as the internal control. Quantitative analysis was done using LabWorks Image Acquisition and Analysis software.

**Statistical analysis:** Quantitative data are expressed as means plus or minus standard error of mean. Statistical analyses were performed using one way ANOVA with Bonferroni post-tests or Kruskal-Wallis with Dunn's post-test where applicable, with p < 0.05 considered significant.

### Results

### CMG response to LPs, BoNT-A and Lipotoxin pretreatment:

The CMG parameters in the LPs (control group), BoNT-A, and Lipotoxin group during intravesical saline instillation at day 1 were not significantly different from that at day 8. These results indicate that the bladder function at day 8 under normal condition was not affected by the pretreatment of LPs, BoNT-A, and Lipotoxin. At day 8, the irritative effect of acetic acid was evident 20 to 30 min after the start of infusion. In LPs and BoNT-A pretreated groups, intercontraction interval (ICI) was significantly decreased by 57.2% and 56.0% respectively after intravesical instillation of AA. However, rats which received prior Lipotoxin treatment showed a significantly reduced response (ICI 21.1% decrease) to AA instillation. These results indicate that Lipotoxin pretreatment suppressed AA induced bladder overactivity, which effect was not seen at identical dose of BoNT-A pretreatment group.

**Histological response to LPs, BoNT-A and Lipotoxin pretreatment:** AA instillation induced moderate inflammatory reaction in LPs and BoNT-A pretreated groups, as determined by the histopathological evaluation of tissue section stained with hamatoxylin and eosin. However, the AA induced inflammatory reaction was significantly decreased in the Lipotoxin pretreated group (total inflammatory score decreased by 48.9% and 33.3% relative to the LPs and BoNT-A pretreated group, respectively). These results indicate that Lipotoxin pretreatment inhibits AA induced bladder inflammation.

**CGRP immunostaining on LPs, BoNT-A and Lipotoxin pretreatment:** CGRP immunostaining was confirmed at the bladder mucosal layer in the Lipotoxin pretreated group, which was not observed in the LPs or BoNT-A pretreated group. These results suggest that Lipotoxin pretreatment inhibits CGRP release.

**SNAP-25 expression on LPs, BoNT-A and Lipotoxin pretreatment:** SNAP-25 positive neuronal fibers were detected in the bladder samples of LPs and BoNT-A pretreated animals. However, SNAP-25 positive neuronal fibers were rarely seen in the Lipotoxin pretreated animals. Western blotting demonstrated that mean SNAP-25 protein level was 66.4% decrease and 58.1% decrease compared to the LPs and BoNT-A pretreated group, respectively. These results indicate that Lipotoxin pretreatment decreased SNAP-25 expression.

### Conclusions:

Intercontraction interval (ICI) was 57.2% and 56.0% decreased after intravesical instillation of AA in the LPs and BoNT-A pretreated rats, respectively. However, rats which received Lipotoxin showed a significantly reduced response (ICI 21.1 % decrease) to AA instillation. In addition, Lipotoxin pretreated rats had a significant decrease in inflammatory reaction and SNAP-25 expression and increase in CGRP immunoreactivity compared with LPs or BoNT-A pretreated rats. Intravesical Lipotoxin administration cleaved SNAP-25 and inhibited CGRP release from afferent nerve terminals, and blocked the AA-induced hyperactive bladder. These results support the LPs as an efficient vehicle for delivering of BoNT-A without the need for injection and avoid effect on the detrusor.

The major findings of the present study are that intravesical Lipotoxin pretreatment suppressed AA induced bladder hyperactivity and inflammatory reaction, which effects were not observed in the LPs and BoNT-A pretreated groups in this animal model. Urinary retention was not seen. Furthermore, the expression of SNAP-25 was significantly reduced and CGRP was significantly increased in the Lipotoxin pretreated group compared to the LPs and BoNT-A pretreated groups in this model. Intravesical Lipotoxin instillation may provide a simpler and effective method for delivering BoNT-A without the need for injection that may cause urinary retention.
1. A liposomal formulation comprising botulinum toxin in a pharmaceutically acceptable carrier for instillation into the bladder.
2. The formulation of paragraph 1 wherein the lipid to botulinum toxin ratio is adjusted to optimize delivery.
3. A method for treating overactive bladder and lower urinary tract symptoms comprising instilling in the bladder botulinum toxin in a liposomal carrier.
4. The formulation of paragraph 1 or method of paragraph 3 wherein the botulinum toxin is one or more of the seven serotypes and double or single chain.
5. The formulation of paragraph 1 or method of paragraph 3 wherein the dose of the botulinum toxin is less than the dose administered by injection to obtain the same effect on overactive bladder and lower urinary tract symptoms,
6. The formulation of paragraph 1 or method of paragraph 3 wherein the volume of the formulation that is instilled is optimized to optimize uptake.
7. The formulation of paragraph 1 wherein the formulation is instilled into other body cavities and surfaces including the vagina, anus, rectum, skin, throat, oral, nasal cavity and airway.

## Claims

1. A liposomal formulation comprising botulinum toxin suitable for instillation into the bladder wherein the botulinum toxin to lipid ratio is up to 0.5 mg lipid per 1 IU of toxin.

2. The formulation of claim 1 wherein the volume of the formulation is effective to provide uptake of a therapeutically effective amount of botulinum toxin across the urothelium.

3. The formulation of claim 2, wherein the volume is between 30 and 60 ml.

4. The formulation of any preceding claim wherein the liposome is prepared by rehydration after freeze drying using an aqueous solution comprising botulinum toxin.

5. The formulation of claim 4 wherein mannitol is added, as a cryoprotectant, at a concentration of 0.5%, 1%, 2.5% or 5% (w/v) prior to freezing.

6. The formulation of claim 4 wherein free botulinum toxin is removed from botulinum toxin entrapped in liposomes.

7. The formulation of any preceding claim wherein the liposomes of the liposomal formulation comprise dipalmitoyl phosphatidylcholine.

8. The formulation of any preceding claim wherein the botulinum toxin is one or more of the seven serotypes and is a double or single chain.

9. The liposomal formulation of any of claims 1 to 8 for use in medicine.

10. The liposomal formulation of any of claims 1 to 8 for use in the treatment of a patient with interstitial cystitis, painful bladder syndrome, overactive bladder, bladder cancer, prostate cancer and urinary tract infections caused by bacteria, fungus or viruses, wherein the liposomal formulation is instilled in the bladder.

11. The liposomal formulation for use of claim 10, wherein the bladder undergoes hydrodistension at the same time as administration of the liposomal formulation.

12. The liposomal formulation for use of claim 10, wherein the volume of the formulation is optimised for uptake of the toxin across the urothelium of the patient.

13. Use of the liposomal formulation of any of claims 1 to 8 in the manufacture of a medicament for the treatment of a patient with interstitial cystitis, painful bladder syndrome, overactive bladder, bladder cancer, prostate cancer and urinary tract infections caused by bacteria, fungus or viruses, wherein the liposomal formulation is instilled in the bladder.

14. The use of claim 13, wherein the bladder undergoes hydrodistension at the same time as administration of the liposomal formulation.

15. The use of claim 13, wherein the volume of the formulation is optimised for uptake of the toxin across the urothelium of the patient.
